# EUROPEAN PATENT SPECIFICATION

(11) **EP 2 672 945 B1**
(45) Date of publication and mention of the grant of the patent: **03.02.2016**
(21) Application number: 11702988.4
(22) Date of filing: 09.02.2011
(51) Int. Cl.: A61J 1/00, A61J 1/03

(54) **STABILIZED PACKAGE FORMS OF SEVELAMER**
STABILISIERTE VERPACKUNGSFORMEN VON SEVELAMER
FORMES D'EMBALLAGE STABILISÉES DE SEVELAMER

(43) Date of publication of application: 18.12.2013
(73) Proprietor: Synthon BV, 6545 CM Nijmegen (NL)
(72) Inventor: BAKKER-HOLMDAHL, Lisa, NL-6545 CM Nijmegen (NL)
(74) Representative: van der Sterren-Mol, Josephine
(86) International application number: PCT/EP2011/051859
(87) International publication number: WO 2012/107083

(56) References cited:
- WO-A1-2006/008651
- WO-A2-2008/062437
- US-A1- 2010 189 595
- US-A1- 2010 300 925
- Dr. Keith Murphy: "Are Nitrogen Molecules Really Larger Than Oxygen Molecules? The correct answer, with respect to "permeation", is yes.", , 1 August 2007 (2007-08-01), XP002660866, Retrieved from the Internet: URL:http://www.getnitrogen.org/pdf/graham. pdf [retrieved on 2011-10-06]

## Description

The present invention relates to a method and package form for insuring stability of organoleptic properties of pharmaceutical compositions comprising the active pharmaceutical ingredient sevelamer.

### BACKGROUND OF THE INVENTION

Sevelamer is a non-absorbed phosphate binding polymer used in the treatment for the control of serum phosphorus in patients with chronic kidney disease (CKD). It is a product of a crosslinking of polyallyl amine with epichlorohydrine and its chemical structure is as follows:

| | |
|---|---|
| 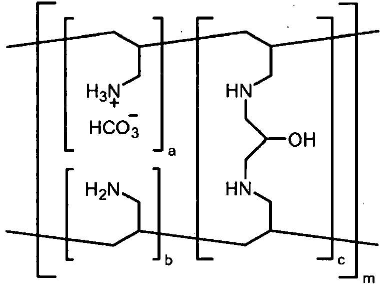 | a, b = number of primary amine groups (a + b = 9) c = number of crosslinking groups (c = 1) m = large number to indicate extended polymer network |

The compound contains multiple amines that become partially protonated in the intestine and interact with phosphate ions through ionic and hydrogen bonding. By binding phosphate in the gastrointestinal tract facilitating phosphorus excretion in feces, sevelamer lowers the plasma phosphorus concentration. The use of sevelamer and its pharmaceutical compositions, and processes for its preparation are disclosed in WO 2006/008651, EP 0716606, EP 0831857, EP 1133989 and EP 1676581.

Sevelamer may form acid addition salts and is currently marketed, i.a., in a form of tablets for oral administration, either as sevelamer carbonate (Renvela®) and/or as sevelamer hydrochloride (Renagel®). Renvela® film-coated tablets contain microcrystalline cellulose, sodium chloride, zinc stearate, hypromellose and diacetylated monoglycerides as inactive ingredients. Renagel® film-coated tablets contain hypromellose, diacetylated monoglyceride, colloidal silicon dioxide and stearic acid as inactive ingredients.

The therapeutic dose of sevelamer is very high; the marketed tablets of sevelamer comprise 400 or 800 mg of the active substance per tablet and the therapeutic dose may be up to 14 g per day. Accordingly, the marketed tablets are packed in plastic bottles comprising large amounts (30-360 pieces) of tablets; each package form thus may contain from 12 to 288 grams of the active substance, which is an extraordinary high amount in comparison with overall package doses used in pharmaceutical industry.

Nothing is known in the publicly available prior art documents about the long-term stability of sevelamer tablets. Apparently, the marketed tablets of sevelamer fulfill general criteria of stability, i.e. the nature and amount of side products and degradation products are within the limits that allow safe administration of the medicinal product by the patient throughout the whole approved shelf-life of the product.

Nevertheless, it was observed by the present inventor that batches of sevelamer tablets, when packed in a plastic container and tested for long-term stability, particularly at an elevated temperature and environmental moisture content, often exhibit an unpleasant smell at opening the container after a certain time. Whereas the prior art is aware of the importance of controlling moisture, the problem of smell has not been addressed in the prior art albeit it appears that it is inherent to most sevelamer tablet compositions to some extent. Thus, without wishing to be bound by any theory, the inventor speculates that the smell is a result of a slow process of degradation related to the sevelamer polymer, not observable by conventional methods. Such process is probably not dangerous to the patient; however, the resulting smell definitely affects the patient's comfort at each opening of the plastic container.

Thus, there exists a need of a certain improvement in the art. In particular, there exists an objective need of stabilization and/or improvement of organoleptic properties of the medicinal product comprising sevelamer, particularly in terms of odour; such stabilization, albeit not affecting the activity and safety of the drug, will prevent potential patient's discomfort when using the medical product, particularly when using a medical product that is close to its expiration time.

### SUMMARY OF THE INVENTION

As an exemplary embodiment, there is provided a package form for storing a sevelamer-comprising pharmaceutical composition, preferably a plurality of tablets, wherein said package form comprises
- a gas-impermeable first container, preferably a plastic container, adapted to contain a sevelamer-comprising pharmaceutical composition, preferably a plurality of sevelamer tablets, more preferably of a size allowing to contain at least 12 g of the active substance of sevelamer within said pharmaceutical composition, and
- a gas-permeable second container adapted to contain a surface active material, preferably silica, such container being in gas contact with the first container, typically being placed inside the first container.

In particular, the second container is a gas-permeable plastic or paper bag and/or it is represented by a separated area in the first container separated from the rest of the first container by a gas-permeable barrier.

In particular, at least 30 sevelamer tablets are placed in the first container.

In particular, each sevelamer tablet comprises at least 400 mg of the active substance, calculated on an anhydrous basis.

Additionally disclosed, there is provided a process for providing a packaged pharmaceutical composition comprising sevelamer, characterized in that a step of filling a gas-impermeable first container with a pharmaceutical composition, typically a plurality of tablets, comprising sevelamer and/or a pharmaceutically acceptable salt thereof, is accompanied with a step of filing a dose of a surface active material into a gas-permeable second container and placing the second container in gas contact with the first container.

The invention relates to the use of a silica gel, for
a] Stabilization of organoleptic properties of pharmaceutical compositions and/or drug forms comprising sevelamer within package forms comprising them; and/or
b] Improving of organoleptic properties of pharmaceutical compositions and/or drug forms comprising sevelamer within package forms comprising them.

### DETAILED DESCRIPTION OF THE INVENTION

Examplary embodiments relate to a package form and a process that are contributory to the stabilization and/or improvement of organoleptic properties, in particular of odour, of sevelamer-containing pharmaceutical compositions, typically tablet compositions.

As discussed above, a problem of unpleasant smell of the content of a container containing sevelamer compositions, particularly tablets, may appear if such a container has been opened after a longer term of storage in a closed state. Apparently, the compounds contributing to the smell are released, albeit in a minimum amount, during the whole period of storage of sevelamer compositions in the container. At a certain moment, their concentration in this limited volume exceeds the threshold detectable by human nostrils and starts affecting the patient's comfort at each opening of the container.

As the nature of the smelling compounds is not known, it is difficult to find a way how to minimize their release during storage. Instead, it now appears more effective to trap them, whenever they are released, into a suitable medium, in which they do not affect the patient's appreciation.

No solution of the problem of minimizing the unpleasant odour of sevelamer-comprising compositions has been provided in the prior art.

It was found that gaseous products causing the unpleasant smell of sevelamer-comprising compositions, typically tablets, in a container may be effectively absorbed and/or adsorbed by a surface active material(s). It was also found out that, in an advantageous way, the surface active material needs not to be placed in a direct contact with the sevelamer composition, but it may be placed in a separated, however sufficiently gas-permeable, second container. Thereby, e.g., a contamination of the surface of the sevelamer-comprising medicament by the surface active material may be prevented.

Thus, there is disclosed a package form for storing a pharmaceutical composition comprising sevelamer and/or its pharmaceutically acceptable salt, typically hydrochloride and/or carbonate (hereinunder often just referred to as sevelamer) and, more typically, a plurality of sevelamer-comprising tablets, wherein such package form minimizes the patient's discomfort associated with an unpleasant odour eventually released during storage. The package form comprises a gas-impermeable first container adapted to contain a sevelamer-comprising pharmaceutical composition, preferably a plurality of sevelamer tablets, and a gas-permeable second container adapted to contain a surface active material, such container being in gas contact with the first container. The "gas-contact" means that the gaseous environment of the first container can freely penetrate into gaseous environment of the second container, and *vice versa*. Typically, the second container is placed inside the first container.

The first container, in which the sevelamer-comprising composition is placed, is, in general, a glass or a plastic vial equipped with a suitable cup or lid, e.g. a screw cup. The plastic material of the container may be a polyolefine, e.g. a polyethylene, polypropylene etc., an ethylene/vinyl acetate copolymer, polyethylene terephthalate, polyvinylchloride etc., inclusive of multi-layer films comprising such materials and/or aluminum or silicon-dioxide lamination placed on the surface of the container. The size of the container is typically such that at least 30 sevelamer-comprising tablets may be placed therein; for instance, 30, 60, 90, 120, 150, 180, 240 and, as in a certain embodiment, 360 tablets are placed therein. The tablets may comprise at least 400 mg of the active substance, calculated on an anhydrous basis, typically 400 mg or 800 mg of the active substance. Thus, the size of the container is typically such that it may contain at least 12 g of sevelamer within a pharmaceutical composition. The active substance may be sevelamer and/or its pharmaceutically acceptable salt, typically hydrochloride and/or carbonate.

Next, the package form also comprises a gas-permeable second container, being in gas contact with the first container and typically placed inside the first container, and comprising a surface active material therein. The content of the second container is fully separated from direct contact with the content of the first container, however, the material from which the smaller container is made has to at least through a certain percentage of the surface of the container allow the gaseous environment of the first container to penetrate into the smaller container and *vice versa*. Thus, gaseous products, whenever present and/or released in the space of the first container, may also reach the space of the second container and contact the content thereof.

Typically, the second container is a paper or plastic bag filled with the surface active material and placed inside the first container. The plastic material, from which the bag is made, is typically a gas-permeable polymer including, but not limited to, polyethylene, polypropylene, polyamide, polyvinylchloride. The bag may be also made from a gas-impermeable material, however, a certain percentage of the bag surface must comprise a gas-permeable "window", through which the equilibration of the gaseous environment in both containers may be assured.

In an alternate arrangement, the surface material may be placed in a suitably separated part of the first container. Typically, such surface active material may be placed in an area under the screw cap and locked there by means of a gas-permeable barrier, e.g. a paper or plastic foil. In another possible arrangement, the surface active material may be placed on the bottom of the first container and then covered by a gas-permeable barrier, which would then form the new bottom of the first container. Again, "gas-permeable" means herein that at least a certain portion of the surface of the barrier is gas-permeable, while the rest may be gas-impermeable. For instance, a barrier made of an otherwise gas-impermeable material may comprise a certain amount of apertures covered by a thin foil of a gas-permeable material. The material assuring the permeation of gases within the container may be any material allowing so; however, preferably, such material is pharmaceutically acceptable. For instance, such material may be the same as that of the plastic foil useful for making bags and as listed above.

The "surface active material" useful in products and processes of the present invention is a material/agent that can absorb or adsorb molecules in the gaseous phase and is limited to a silica gel unclaimed alternatives include an aluminum silicate; a alkali-metal and/or alkaline earth metal aluminosilicate; a zeolite; an activated carbon; or a combination thereof.

The amount of the surface active material to be placed in the second container depends on the overall amount of sevelamer present in the pharmaceutical composition placed in the first container. Typically, 0.1 g to 5 g, more preferably 0.5 g to 4 g, of the surface active material per 100 g of sevelamer may be used.

The package form for storing a pharmaceutical composition comprising sevelamer, typically sevelamer-comprising tablets, may be made by a process characterized in that a step of filling a gas-impermeable first container with a composition, typically a plurality of tablets, comprising sevelamer and/or a pharmaceutically acceptable salt thereof, is accompanied with a step of filing a dose of a surface active material into a gas-permeable second container and placing the second container in gas contact with the first container.

The order of these two steps is essentially irrelevant.

In a special aspect, the second container with the surface active material may be added to the content of the first container considerably later than filing of the first container. By doing this, the already pronounced odour of the content of the first container may even be eliminated. Thus, the surface active material may be added to packed sevelamer tablets whenever during the storage life-time.

The second container comprising the surface active material may be prepared and filled quite independently of the filling of the first container, however, it has to be stored prior to placing it into contact with the environment of the first container under conditions preventing loss of its activity. In particular, the surface active material has to be stored protected from humidity.

With respect to the nature and material of the containers, the way of assembling both containers together, the size of both containers and the nature and amount of the surface active material, reference is made to the data published above.

Thus, in association with the product and process disclosed above, the present invention relates to the use of a surface active material, for
a] stabilization of organoleptic properties of pharmaceutical compositions comprising sevelamer within package forms comprising them; and/or
b] improving of organoleptic properties of pharmaceutical compositions comprising sevelamer within package forms comprising them.

Thus, the surface active material may be used not only for long-term protection of packed sevelamer compositions, e.g., tablets, against an unpleasant odour that may appear during prolonged storage, but also for elimination of such odour after it already appeared. This means that the surface active material of the present invention may be used not only in combination with freshly prepared and packed sevelamer compositions, but also with compositions packed and stored for a certain time, after such odour has already been registered by the user.

The best mode of practicing the present invention as well as the results of experiments showing the superiority of the products and processes of the present invention are summarized in the following Examples.

### EXAMPLES

### Example 1

Several batches of sevelamer-comprising coated tablets of the formulae below were made according to the following process.

Sevelamer and colloidal silicon dioxide were blended (Turbula blender, 5 minutes) and screened. Remaining excipients - except lubricant - were added and blended (15 minutes). Lubricant was added for final blending (5 min). Blend was compressed into tablets with 30 kN compression force using Excenter Press (Korsch EK-0).

The tablet cores were coated in a conventional drum coater.

| Ingredient | A | B | C | D | E |
|---|---|---|---|---|---|
| | weight (mg) | weight (mg) | weight (mg) | weight (mg) | weight (mg) |
| *core* | | | | | |
| Sevelamer carbonate | 381.0 | 381.0 | 381.0 | 381.0 | 381.0 |
| Microcrystalline cellulose | 50.0 | 50.0 | 50.0 | 50.0 | 50.0 |
| Silicified MCC | 64.0 | 64.0 | 64.0 | 64.0 | 64.0 |
| Colloidal silicon dioxide | 2.5 | 2.5 | 2.5 | 2.5 | 2.5 |
| Zinc stearate | 2.5 | 2.5 | 2.5 | 2.5 | 2.5 |

| *coating* | | | | | |
|---|---|---|---|---|---|
| Opadry 85F18422 white | 22.5 | | | | |
| Opady85F19250 clear | | 19.0 | 42.0 | | |
| IPMC E5/E15 + triacetin | | | | 24.5 | 50.5 |

The respective batches were filled (with 30 tablets) in closed high-density polyethylene DUMA® containers and stored for 6 months at 40°C and 75% of relative humidity. A part of the containers were filled with an inert gas (nitrogen or carbon dioxide) and closed, to another part of the bottles a lid with 2 g of silica gel was added.

After opening the containers, the odour of the tablets was evaluated organoleptically (no reliable objective tests are disclosed in Pharmacopoeias). The results are shown in the following table.

| Batch | Package | Smell |
|---|---|---|
| A | HDPE | Pungent odour |
| | HDPE + nitrogen | Pungent odour |
| | HDPE + carbon dioxide | Pungent odour |
| | HDPE + silica | No smell |
| B | HDPE | Pungent odour |
| | HDPE + nitrogen | Pungent odour |
| | HDPE + carbon dioxide | Pungent odour |
| | HDPE + silica | No smell |
| C | HDPE | Pungent odour |
| | HDPE + nitrogen | Pungent odour |
| | HDPE + carboy dioxide | Pungent odour |
| | HDPE + silica | No smell |
| D | HDPE | Pungent odour |
| | HDPE + nitrogen | Pungent odour |
| | HDPE + carbon dioxide | Pungent odour |
| | HDPE + silica | No smell |
| E | HDPE | Pungent odour |
| | HDPE + nitrogen | Pungent odour |
| | HDPE + carbon dioxide | Pungent odour |
| | HDPE + silica | No smell |

### Example 2

Sevelamer tablets of the following composition were prepared by a process as described in Example 1.

| | |
|---|---|
| Sevelamer carbonate | 800 mg |
| Lactose monohydrate | 250.25 mg |
| Colloidal silicon dioxide | 5.55 mg |
| Zinc stearate | 8.25 mg |

To a batch of 180 tablets filled in a closed HDPE bottle and stored already for 12 months at ambient conditions, a lid containing 2 g of silica gel was added.

While the content of bottles originally exhibited pungent odour, after 5 days in contact with the lid the odour had completely disappeared.

The invention having been described, it will be readily apparent to those skilled in the art that further changes and modifications in actual implementation of the concepts and embodiments described herein can easily be made or may be learned by practice of the invention, without departing from the scope of the invention as defined by the following claims.

## Claims

1. Use of a silica gel, for stabilization of organoleptic properties of pharmaceutical compositions comprising sevelamer within package forms comprising them.

2. Use of a silica gel, for improving of organoleptic properties of pharmaceutical compositions comprising sevelamer within package forms comprising them.

## Patentansprüche

1. Verwendung eines Silikagels zur Stabilisierung von organoleptischen Eigenschaften pharmazeutischer Zusammensetzungen umfassend Sevelamer innerhalb von Verpackungsformen, die sie umfassen.

2. Verwendung eines Silikagels zum Verbessern von organoleptischen Eigenschaften pharmazeutischer Zusammensetzungen umfassend Sevelamer innerhalb von Verpackungsformen, die sie umfassen.

## Revendications

1. Utilisation d'un gel de silice, pour stabiliser les propriétés organoleptiques de compositions pharmaceutiques comprenant du sévélamer dans des formes de conditionnement les comprenant.

2. Utilisation d'un gel de silice, pour améliorer les propriétés organoleptiques de compositions pharmaceutiques comprenant du sévélamer dans des formes de conditionnement les comprenant.
